# EUROPEAN PATENT APPLICATION

(11) **EP 1 872 802 A1**
(43) Date of publication of application: **02.01.2008**
(21) Application number: 07252636.1
(22) Date of filing: 28.06.2007
(51) Int. Cl.: A61L 2/18, A47K 5/12, G08B 21/24, G06F 19/00

(54) **Hand washing compliance system**

(30) Priority: 29.06.2006 US 427458
(71) Applicant: ETHICON, INC., Somerville, New Jersey 08876 (US)
(72) Inventor: Raja, Vishnu R., Irvine CA 92614 (US); Lin, Szu-Min, Irvine CA 92620 (US); Platt, Robert, Laguna Niguel CA 92677 (US); Jacobson, Brent, Newport Coast CA 92657 (US); Hagerman, Josh, Oceanside CA 92056 (US); Heniges, Steve, Carlsbad CA 92009 (US)
(74) Representative: Fisher, Adrian John

(57) **Abstract**

A system and method for evaluating hand decontamination compliance in a medical care facility includes a plurality of patient stations; a plurality of hand hygiene stations; personnel tags for personnel in the medical care facility; readers at the patient stations and at the hand hygiene stations capable of detecting the presence of personnel tags; and a controller for detecting whether a personnel tag accessed a hand decontamination station before presence at one of the patient stations. In an alternate embodiment, personnel tags of healthcare workers and patients are tracked in space within the facility to indicate contacts between the healthcare workers and the patients and whether the healthcare workers accessed hand hygiene prior to the contact. Hand hygiene stations include hand washing stations and antimicrobial hand rub dispensers.

## Description

### Background of the Invention

This application relates to monitoring of hand washing and decontamination, and in particular monitoring of hand washing and decontamination in a healthcare environment.

Acquisition of infection by hospital patients is a serious healthcare problem. The Center for Disease Control, the World Health Organization and other health care organizations and agencies encourage health care workers to practice proper hand hygiene to reduce the transmission of pathogens via hands. Recommended procedures include the decontamination of the hands prior to direct patient contact, prior to invasive non-surgical procedures, prior to gloving, after contact with body fluid, mucous membranes, non-intact skin and wound dressings, intact skin and inanimate objects near patients. These procedures apply in hospital settings, doctor's offices, and anywhere where these personnel come into contact with patients. Furthermore, it is generally recommended that visitors to such patients also practice proper hand washing procedures. In some instances, application of an antimicrobial preparation to the hands is substituted for a hand washing. In any event, the goal is to reduce the microbe load on the healthcare provider's hands and prevent contamination of either the patients or healthcare providers.

### Summary of the Invention

A system, according to the present invention, provides for evaluating hand hygiene compliance in a medical care facility,. The system comprises a plurality of patient stations; a plurality of hand hygiene stations; personnel tags for personnel in the medical care facility; readers at the patient stations and at the hand decontamination stations capable of detecting the presence of personnel tags; and control means for detecting whether a personnel tag accessed a hand decontamination station before presence at one of the patient stations.

The patient stations typically would comprise beds, but also can include procedure tables, examination tables, dentist chairs and any other location where interactions occur between patients and healthcare workers.

Preferably, each patient station will have a unique patient station identifier and each personnel tag will have a unique personnel identifier, making possible the tracking of specific patient healthcare worker interactions. Individual hand hygiene stations can also bear unique identifiers. If a hand hygiene station were located too long of a distance from patient station presence there may not be considered sufficient for a subsequent patient interaction a the patient station.

Hand hygiene stations can include hand washing stations, such as a sink with a soap dispenser, can also include anti-microbial hand rub dispensers. Preferably, they also include an actuation sensor for sensing not just presence but actual use of the hand hygiene station, the actuation sensor being linked to the control means.

The personnel tags comprise a machine readable sensor such as an RFID tag. Hand hygiene status of a user bearing the personnel tag is preferably stored on the personnel tag itself but can also be stored on a central processor linked to multiple patient stations and one or more hand hygiene stations. The central processor can generate reports of compliance rates. Such reports might be averaged for the facility or specific to individual healthcare workers. They can include the number of patient interactions, the number of patient interactions where proper hand hygiene was practices the number of interactions where proper hand hygiene was not practiced and whether proper hand hygiene was practiced after a patient interaction.

Preferably, the patient stations incorporate some form of alarm which can provide a warning if a personnel tag having a status other than "clean" approaches the patient station. The warning can be both audible and visual. Preferably, means are provided for defining a perimeter about the patient station within which the personnel tag is considered present at the patient station.

Preferably, the personnel tags comprise a status indicator to indicate the hand hygiene status of its bearer. Such a status indicator can provides a visual indication of the hand hygiene status and also provide an audible indication of a change in status, or an audible warning of improper patient contact.

A method according to the present invention provides for evaluating hand hygiene compliance in a medical care facility. The method comprises the steps of: tagging personnel in such facility with a personnel tag; detecting presence of personnel tags at hand hygiene stations within the facility; detecting presence of personnel tags at patient stations; and determining whether a person tagged with a personnel tag has visited a hand hygiene station prior to visiting a patient station.

The method preferably stores a hand hygiene status of a user, such as "clean" or "potentially contaminated." This status changes from "clean" after contact with a patient. It will also change from "clean" after a predetermined time regardless of patient contact.

An alternative system, according to the present invention, provides for evaluating hand hygiene compliance in a medical facility. The system comprises: personnel tags for patients and for healthcare workers within the facility; sensors for determining a location of the personnel tags; and a controller programmed to detect whether a healthcare worker bearing one of the personnel tags accessed a hand hygiene station prior to contacting a patient bearing a separate one of the personnel tags. Hygiene tags can be provided at the hand hygiene stations within the facility. Alternatively, their locations can be programmed in. Tags are beneficial for stations which might be mobile.

### Brief Description of the Drawings

FIG. 1 is a diagram view of a hand hygiene compliance system according to the present invention;
FIG. 2 is a plan view of a personnel tag according to the present invention; and
FIG. 3 is a diagram view of an alternative embodiment of a hand hygiene compliance system according to the present invention.

### Detailed Description of the Preferred Embodiment

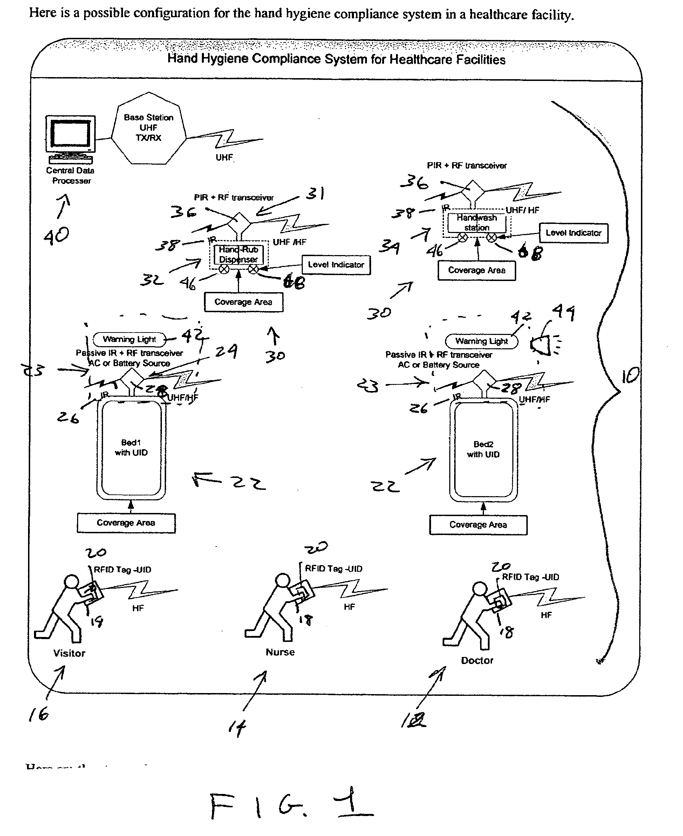

FIG. 1 illustrates a hand hygiene compliance system 10 for a healthcare facility. Persons within the facility such as a doctor 12, nurse 14 or visitor 16 are each provided with a personnel tag 18, which preferably incorporates a unique identifying number but may not for privacy reasons. The tag 18 preferably incorporates some form of proximity locator or local communication means. For instance, in a preferred embodiment of the invention, the tag 18 incorporates a Radio Frequency IDentification (RFID) tag 20.

Patient contact locations such as hospital bed 22 are provided with a patient location module 23 comprising sensors 24 to detect the approach of persons such as a nurse 14 or the approach of the tag 18. Multiple sensors 24 and a sensors of different types could be employed. For instance, a passive infrared radiation (IR) sensor 26 which detects the approach of a person can be employed along with an RFID transceiver 28 for reading the RFID tag 20 in the personnel tag 18. This would allow the RFID transceiver to remain off until the bed 22 is approached, saving energy and reducing electromagnetic noise in the environment.

Some form of perimeter is preferably defined about a patient station. In a simple form it can be the location of the RFID transceiver 28 on the station with the RFID transceiver having a range equivalent to the desired perimeter. The perimeter is preferably about one or two feet from the patient station. If it is too far it might falsely register a contact and if it is too small it might fail to register a contact. Other means for defining a perimeter such as light beams broken via personnel approaching a patient station may also be employed if a more exactly defined perimeter is desired.

Other machine readable tagging systems may be employed. RFID tags 20 are particularly suitable for this application as they are inexpensive, can be read at a distance and some types can have data written to them and updated.

The patient contact location is not limited to beds, but might also include procedure tables, examination tables, dental chairs, gurneys and any location at which a health care worker is likely to come into contact with a patient. These locations may be mobile, such as gurneys.

Hand hygiene stations 30 (such as an antimicrobial hand rub dispenser 32 or hand wash station 34 such as a sink with running water and a supply of soap or detergent for hand washing) are provided with hand hygiene station modules 31 having an RFID transceiver 36. US Patent No. 5,997,893 to Jampani et al., incorporated herein by reference, discloses a particularly suitable anti-microbial hand rub formulation.

Preferably, the RFID transceivers 28 and 36 are all tied through some form of communication to a central data processing station 40. That communication could take the form of a hard-wired connection or some other form such as radio frequency communication. In an institution having an 802.11b or 802.11g or similar type of WiFi based networking or communication system the RFI transceivers 28 and 36 could communicate to the central processing station 40 through such network. A dedicated communication system could also be employed. Passive IR personnel detectors 38 can be employed at the hand hygiene stations 30 as well.

Hand hygiene stations 30, especially hand rub dispensers 32 can be mobile. They can be mounted on wheels or carried on a person such as is disclosed in U.S. Patent No. 5,927,548 to Villaveces, incorporated herein by reference.

In its basic operation, the hand hygiene compliance system 10 employs the RFID transceivers 28 and 36 along with the personnel tags 18 to help ensure that a person who approaches a patient location has either washed their hands or applied an antimicrobial hand rub to reduce the chance of infecting a patient. This can be implemented in many different fashions. One simple implementation would detect the person's presence at a hand hygiene station 30 and then signal either to the RFID tag 20 or to the central processor 40, or to both, that the person's status was now "clean." Then when that person approached a hospital bed 22 the RFID transceiver 28 associated therewith would read the RFID tag 20 in the personnel tag 18. If the status was directly encoded within the RFID tag 20 it would be read directly by the RFID transceiver 28, otherwise a unique ID associated with that RFID tag 20 would be queried back to the central processor 40 which would return the status "clean."

Preferably, the patient location module 23 incorporates a display 42 of some fashion with an audio output device 44. The display 32 could be as simple as one or more color-coded lights, preferably labeled, or more complex such as an LCD panel. Therefore, when the person approached the patient bed 22 the display 42 would indicate such as by a green light or display of the status "clean" that the person had attended to hand hygiene prior to visiting that hospital bed 22.

If the status is not "clean" but is rather "potentially contaminated" then the patient location module 23 will indicate a warning. Preferably, the warning would include either a warning light or a warning message on the display 42 and an audio alert such as a buzzer or more preferably voice instructions to attend to hand hygiene.

After leaving the patient bed 22, the RFID tag 20 would now have the status "potentially contaminated". The status "potentially contaminated" would also apply when there had not been previous contact with a hand hygiene station 30. Status would be changed back to "clean" upon visitation of a hand hygiene station 30. The "clean" status would be effective for a specified period of time assuming there is no further contact with patient locations. In more elaborate implementations of the invention sensors such as the RFID transceiver 28 could be placed in other locations within a facility which could be possible sources of contamination and contact therewith would change a status from "clean" to "potentially contaminated".

Methods are potentially included to allow a personnel tag 18 to move away from a patient bed 22 and return to that same bed 22 without having the status register as "potentially contaminated." This would allow, for instance, a nurse 14 to move about a patient's room without having the status change, or at least the status as respect to that particular patient bed 22. Preferably a time limit would be implemented so that even if the nurse 14 re-approaches the bed 22 after that time limit the status registers as "potentially contaminated."

Other implementations and data storage can be included within the invention. For instance, compliance rates for various personnel can be tracked. The tracking can be performed on the RFID tag 20 itself or at the central processor 40. Reports can be generated and used to help personnel improve their compliance. Such reports might include the number of times such personnel approached a patient location with a status other than "clean" within a given time period. It could also track which patients were approached and be compared against patient records to track transmission of infections within the institution. Penalties or bonus can be awarded personnel based upon compliance.

If a doctor 12 or nurse 14 approach a patient bed 22 in a condition other than "clean" the system 10 preferably checks whether such person then proceeds to a hand hygiene station 30 or continues with patient contact. An exception in this area may be flagged with a higher priority by the system 10, and can be identified by comparing time present at the bed 22, time to the next appearance at a hand hygiene station 30 and possibly whether such personnel then return to the same bed 22. For instance, spending more than five or ten seconds at the bed 22 in a state other than "clean" might constitute an egregious violation.

Compliance at a hand hygiene station 30 can either be assumed by presence, assumed by presence for a given amount of time or verified with a sensor 46 at the hand hygiene station 30 such as a sensor 30 which reads when soap is dispensed at a hand wash station 34 or a sensor 30 which reads when an antimicrobial hand gel has been dispensed at a hand rub dispenser 32. Such sensors 46 would be important when the hand hygiene station 30 comprises a portable antimicrobial hand rub dispenser 32 worn on the body of the user.

Hand hygiene procedures typically require a certain length of scrubbing at hand wash stations 34 and the time of water running after dispensing of the soap might also be measured. A proximity sensor, especially one already used to turn on water flow, might also be polled to see if hands are in the stream of the water. Many faucets already incorporate such sensors.

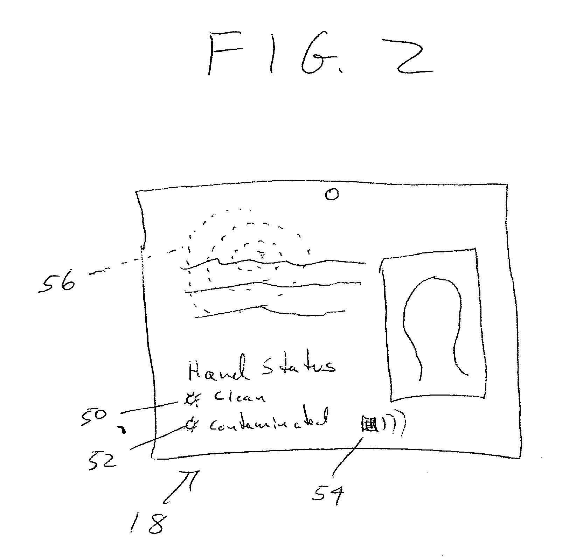

The personnel tags 18 can further incorporate audio and visual displays. For instance it might bear a light which changes color or separate lights 50 and 52 for each status and a beeper 54 which emits a tone upon a change in status (see FIG. 2). In such instance they preferably incorporated powered RFID tags 56 which have improved communication ranges over passive RFID tags and a common power source could power all of the features of such tags 18.

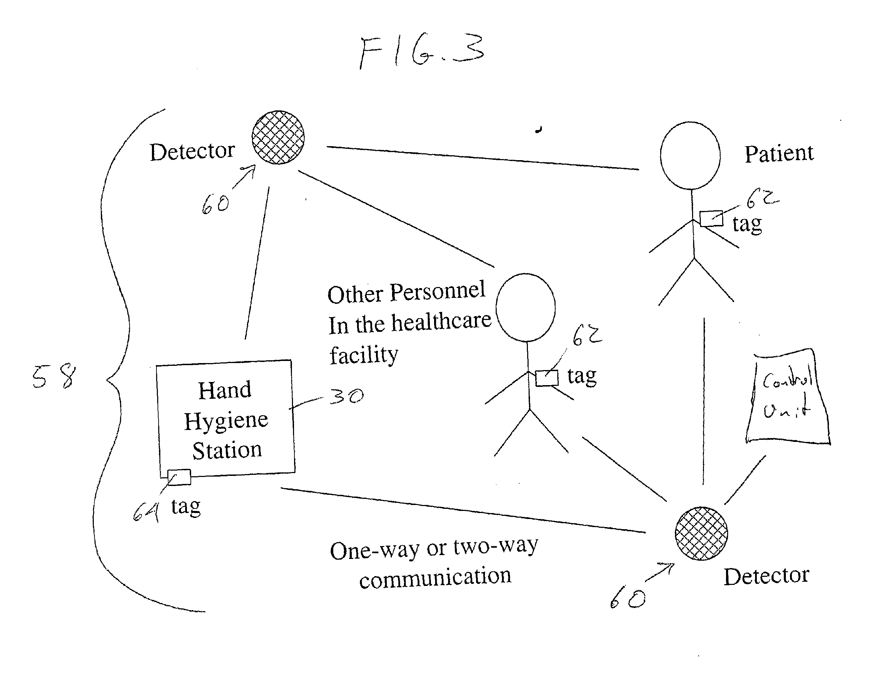

Turning also to FIG. 3, an alternative embodiment of a hand hygiene compliance system 58 would employ multiple detectors 60 throughout a facility which would read and located personnel tags 62. In such a system 58, a person's exact location within a facility would be tracked. Patients also would have personnel tags 62. With such a system proximity between personnel tags 62 could be tracked.

If a personnel tag 62 assigned to a doctor 12, nurse 14 or visitor 16 approached a personnel tag 62 assigned to a patient in a status other than "clean" a violation could be registered. Preferably such a tag 62 would incorporate its own warning system such as the audio and visual displays of the tag 18 shown in FIG. 2. Therefore contact away from traditional patient locations such as beds 22 could be tracked. Rather than a transceiver, a hand hygiene station 30 would have its own tag 64, or its location could just be programmed into a central processing controller 66. Proximity to the hand hygiene station 30 would change status to "clean.." The more elaborate procedures mentioned before could also be employed.

In either system the readiness of the hand hygiene station 30 could be tracked. If it is a hand wash station 34 the level of hand soap in a dispenser could be tracked with a level sensor 68. If it is a hand rub dispenser 32 the amount of hand rub could be similarly watched with a level sensor 68, preferably in communication with the central processing controller 40 or 66. Rather than a level sensor 68, if a dispenser 32 were to dispense an accurate amount with each actuation the number of actuations as monitored by the actuation sensor 46 could be counted. Upon emptying a message to refill the dispenser 32 could be generated to appropriate personnel. Shelf life could similarly be monitored, and even incorporate a temperature sensor (not shown) to adjust for temperature effects upon shelf life. Upon expiration of shelf life a warning could be generated to appropriate personnel.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A system for evaluating hand hygiene compliance in a medical facility, the system comprising:
personnel tags for patients and for healthcare workers within the facility;
sensors for determining a location of the personnel tags; and
a controller programmed to detect whether a healthcare worker bearing one of the personnel tags accessed a hand hygiene station prior to contacting a patient bearing a separate one of the personnel tags.

2. A system according to claim 1 and further comprising hygiene tags at one or more hand hygiene stations within the facility.

3. A system according to claim 1 wherein each personnel tag carries a unique identifier.

4. A system according to claim 1 wherein one or more hand hygiene stations comprise hand washing stations.

5. A system according to claim 1 wherein one or more hand hygiene stations comprise anti-microbial hand rub dispensers.

6. A system according to claim 1 and further comprising an actuation sensor at a hand hygiene station for sensing actuation thereof, the actuation sensor being linked to the controller.

7. A system according to claim 1 wherein the personnel tags worn by the healthcare workers comprise a status indicator to indicate a hand hygiene status of the healthcare worker.

8. A system according to claim 7 wherein the status indicator provides a visual indication of the hand hygiene status.

9. A system according to claim 7 wherein the status indicator provides an audible indication of a change in status.

10. A system according to claim 7 wherein the status indicator provides an audible warning when the personnel tag is within a defined proximity of a personnel tag of a patient and the hand hygiene status is other than "clean."

11. A method of evaluating hand hygiene compliance in a medical care facility, the method comprising the step of:
tagging healthcare workers and patients within the facility with a personnel tag;
sensing the physical location within the facility of each such personnel tag; and
determining whether a healthcare worker bearing one of the personnel tags accessed a hand hygiene station prior to contacting a patient bearing a separate one of the personnel tags.

12. A method according to claim 11 and further comprising the step of providing a hygiene tag on the hand hygiene station and determining the physical location of the hand hygiene station within the facility by sensing the location of the hygiene tag.

13. A method according to claim 11 wherein each personnel tag carries a unique identifier.

14. A method according to claim 11 wherein one or more hand hygiene stations comprise hand washing stations.

15. A method according to claim 11 wherein one or more hand hygiene stations comprise anti-microbial hand rub dispensers.

16. A method according to claim 11 and further comprising the step of sensing whether the hand hygiene station was actuated when it was accessed by the healthcare worker.

17. A method according to claim 11 and further comprising the step of displaying a hygiene status of the healthcare worker on the personnel tag.

18. A method according to claim 17 and further comprising the step of providing an audible indication of a change in status.

19. A method according to claim 7 and further comprising the step of providing audible warning when the personnel tag is within a defined proximity of a personnel tag of a patient and the hygiene status is other than "clean."
